# EUROPEAN PATENT APPLICATION

(11) **EP 4 421 067 A1**
(43) Date of publication of application: **28.08.2024**
(21) Application number: 22883504.7
(22) Date of filing: 14.10.2022
(51) Int. Cl.: C07D 231/20, C07D 231/52, C07D 405/12, C07D 417/04, C07D 417/12

(54) **FLUORINE-CONTAINING PYRAZOLE COMPOUND AND PRODUCTION METHOD THEREFOR**

(30) Priority: 19.10.2021 JP 2021171113
(71) Applicant: Unimatec Co., Ltd., Tokyo 105-0012 (JP)
(72) Inventor: SEINO, Junya, Kitaibaraki-shi, Ibaraki 319-1593 (JP); AOTSU, Rie, Kitaibaraki-shi, Ibaraki 319-1593 (JP); KOKIN, Keisuke, Kitaibaraki-shi, Ibaraki 319-1593 (JP)
(74) Representative: Global IP Europe Patentanwaltskanzlei
(86) International application number: PCT/JP2022/038421
(87) International publication number: WO 2023/068200

(57) **Abstract**

Provided is a novel fluorine-containing pyrazole compound having a halogen atom, an oxygen-containing substituent, or a nitrogen-containing substituent at the 3-position of a pyrazole ring, a trifluoromethyl group at the 4-position of the pyrazole ring, and an oxygen-containing substituent at the 5-position of the pyrazole ring, and a production method capable of easily producing the fluorine-containing pyrazole compound. The fluorine-containing pyrazole compound is represented by the following general formula (1): 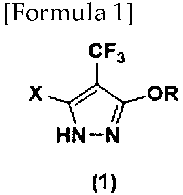 wherein in formula (1) above, R represents a hydrocarbon group having 1 to 12 carbon atoms; X represents a heterocyclic group containing at least one heteroatom selected from the group consisting of a halogen atom, nitrogen and oxygen as a ring atom, -OA¹, or -NA¹A²; and A¹ and A² each independently represent a hydrogen atom or an organic group having 1 to 10 carbon atoms.

## Description

### Technical Field

The present invention relates to a fluorine-containing pyrazole compound and production method therefor.

### Background Art

Conventionally, fluorine-containing pyrazole compounds have been reported to have various biological activities. Among them, a compound having a nitrogen-containing substituent at the 3-position of a pyrazole ring and a methoxy group at the 5-position of the pyrazole ring is expected to be used in the fields of medicine and agrochemicals. Specifically, for a compound having a 3-heteroarylamino-5-methoxy-pyrazole structure, Non Patent Literature 1 reports that it has an inhibitory activity of Janus kinases JAK2 and JAK3, and Non Patent Literature 2 reports that it has an inhibitory activity of tropomyosin receptor kinase A. Moreover, Patent Literature 1 has reported a production method of a compound having a nitrogen-containing substituent at the 3-position of a pyrazole ring, a trifluoromethyl group at the 4-position of the pyrazole ring, and a hydroxyl group at the 5-position of the pyrazole ring.

From the above viewpoints, with an expectation of further improvement in activity, a compound having a halogen atom, an oxygen-containing substituent, or nitrogen-containing substituent at the 3-position of a pyrazole ring, a trifluoromethyl group at the 4-position of the pyrazole ring, and an oxygen-containing substituent at the 5-position of the pyrazole ring has been attracting interest in development thereof.

### Document List

### Patent Literature

Patent Literature 1: Japanese Patent Application Publication No. 61-118371

### Non Patent Literatures

Non Patent Literature 1: Bioorganic & Medicinal Chemistry Letters, Vol. 23, 2013, pp. 3105 to 3110
Non Patent Literature 2: ACS Medicinal Chemistry Letters, Volume 3, 2012, pp. 705 to 709

### Summary of Invention

### Technical Problem

For a compound having a halogen atom, an oxygen-containing substituent, or a nitrogen-containing substituent at the 3-position of a pyrazole ring and an oxygen-containing substituent at the 5-position of the pyrazole ring, no reaction examples introducing a trifluoromethyl group at the 4-position of the pyrazole ring have been reported so far. This is because an introduction step of the trifluoromethyl group raises a problem of controlling reactivity and reaction selectivity of a substrate. As reported in Patent Literature 1, on the other hand, reaction of a compound having a nitrogen-containing substituent on the 3-position of a pyrazole ring, a trifluoromethyl group on the 4-position of the pyrazole ring, and a hydroxyl group on the 5-position of the pyrazole ring with a methylation agent is considered to convert a substituent on the 5-position of the pyrazole ring to a methoxy group. In such a reaction, a methyl group is expected to be introduced at either the 1-position or 2-position of the pyrazole ring, and there has been room for further improvement in terms of reaction efficiency, such as selectivity.

Therefore, the present inventors have discovered that the reaction with a specific raw material enables synthesis of a pyrazole compound having a halogen atom, an oxygen-containing substituent, or a nitrogen-containing substituent at the 3-position of a pyrazole ring, a trifluoromethyl group at the 4-position of the pyrazole ring, and an oxygen-containing substituent at the 5-position of the pyrazole ring, and thus have completed the present invention. That is, an object of the present invention is to provide a novel fluorine-containing pyrazole compound having a halogen atom, an oxygen-containing substituent, or a nitrogen-containing substituent at the 3-position of a pyrazole ring, a trifluoromethyl group at the 4-position of the pyrazole ring, and an oxygen-containing substituent at the 5-position of the pyrazole ring, which has not been known conventionally, and a production method capable of easily producing the fluorine-containing pyrazole compound.

### Solution to Problem

The configuration of gist of the present invention is as follows.
[1] A fluorine-containing pyrazole compound represented by the following general formula (1): 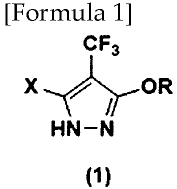
   wherein R represents a hydrocarbon group having 1 to 12 carbon atoms,
   X represents a heterocyclic group containing at least one heteroatom selected from the group consisting of a halogen atom, nitrogen and oxygen as a ring atom, -OA¹, or -NA¹A², and
   A¹ and A² each independently represent a hydrogen atom or an organic group having 1 to 10 carbon atoms.
[2] A method for producing a fluorine-containing pyrazole compound, including reacting a fluoroisobutylene derivative represented by the following general formula (2) with a compound represented by the following general formula (3) or a salt thereof to obtain a fluorine-containing pyrazole compound represented by the following general formula (4): 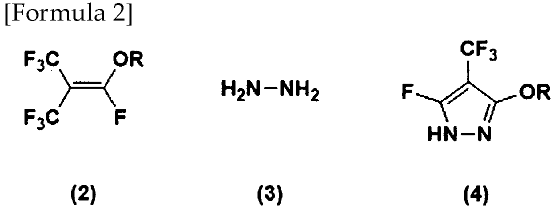 wherein R represents a hydrocarbon group having 1 to 12 carbon atoms.
[3] A method for producing a fluorine-containing pyrazole compound, including reacting a fluoroisobutylene derivative represented by the following general formula (2) with a compound represented by the following general formula (3) or a salt thereof and a compound represented by the following general formula (5) to obtain a fluorine-containing pyrazole compound represented by the following general formula (1): 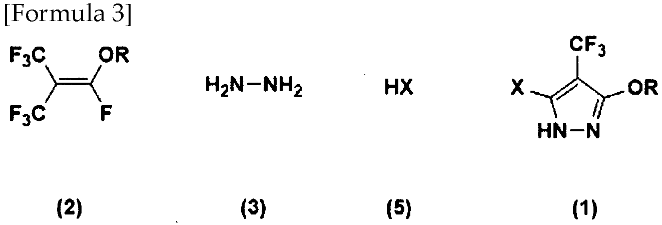
   wherein R represents a hydrocarbon group having 1 to 12 carbon atoms,
   X represents a heterocyclic group containing at least one heteroatom selected from the group consisting of a halogen atom, nitrogen and oxygen as a ring atom, -OA¹, or -NA¹A², and
   A¹ and A² each independently represent a hydrogen atom or an organic group having 1 to 10 carbon atoms.
[4] A method for producing a fluorine-containing pyrazole compound, including reacting a fluoroisobutane derivative represented by the following general formula (6) with a compound represented by the following general formula (3) or a salt thereof to obtain a fluorine-containing pyrazole compound represented by the following general formula (4): 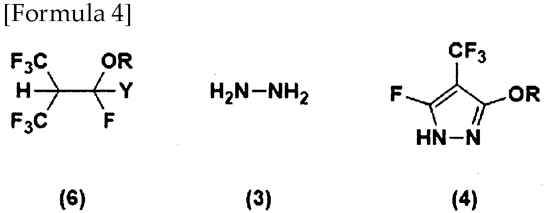
   wherein R represents a hydrocarbon group having 1 to 12 carbon atoms,
   Y represents a halogen atom, -OA³, -SOₘA³ where m is an integer of 0 to 3, or - NA³A⁴, and
   A³ and A⁴ each independently represent a hydrogen atom or a hydrocarbon group having 1 to 10 carbon atoms.
[5] A method for producing a fluorine-containing pyrazole compound, including reacting a fluoroisobutane derivative represented by the following general formula (6) with a compound represented by the following general formula (3) or a salt thereof and a compound represented by the following general formula (5) to obtain a fluorine-containing pyrazole compound represented by the following general formula (1): 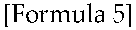 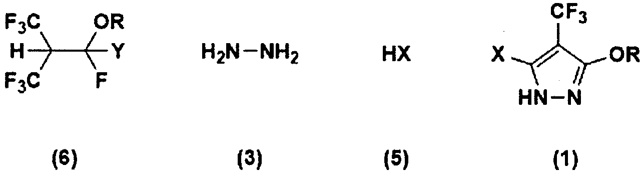
   wherein R represents a hydrocarbon group having 1 to 12 carbon atoms,
   X represents a heterocyclic group containing at least one heteroatom selected from the group consisting of a halogen atom, nitrogen and oxygen as a ring atom, -OA¹, or -NA¹A²,
   A¹ and A² each independently represent a hydrogen atom or an organic group having 1 to 10 carbon atoms,
   Y represents a halogen atom, -OA³, -SOₘA³ where m is an integer of 0 to 3, or - NA³A⁴, and
   A³ and A⁴ each independently represent a hydrogen atom or a hydrocarbon group having 1 to 10 carbon atoms.

### Effects of Invention

A novel fluorine-containing pyrazole compound having a halogen atom, an oxygen-containing substituent, or a nitrogen-containing substituent at the 3-position of a pyrazole ring, a trifluoromethyl group at the 4-position of the pyrazole ring, and an oxygen-containing substituent at the 5-position of the pyrazole ring, and a production method capable of easily producing the fluorine-containing pyrazole compound, can be provided.

### Description of Embodiments

### (Fluorine-containing pyrazole compound)

The fluorine-containing pyrazole compound of the present invention is represented by the following general formula (1): 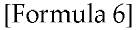 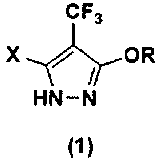
wherein in formula (1) above, R represents a hydrocarbon group having 1 to 12 carbon atoms,
X represents a heterocyclic group containing at least one heteroatom selected from the group consisting of a halogen atom, nitrogen and oxygen as a ring atom, -OA¹, or -NA¹A², and
A¹ and A² each independently represent a hydrogen atom or an organic group having 1 to 10 carbon atoms.

The fluorine-containing pyrazole compound of the present invention has specific substituents (-X, -CF₃, -OR) on the 3-position, 4-position, and 5-position of the pyrazole ring, and thereby it can have an excellent effect from the viewpoint of structural expandability. In particular, desired biological activities (for example, hormone or enzyme inhibitory activity, bactericidal activity, insecticidal activity, and herbicidal activity) can be expected. In particular, examples of the bactericidal activity include the bactericidal activity of bacteria having a harmful effect on agricultural products such as the human body and rice. Moreover, the substituents on the 3- and 5-positions of the pyrazole ring being different groups (-X and -OR) facilitates derivatization into an asymmetric structure by elimination or reaction of these groups, which can be expected to be used as an intermediate. More specifically, a fluorine-containing pyrazole compound being reacted under acidic conditions to modify -OR enables to form a derivative. Moreover, a fluorine-containing pyrazole compound being reacted under basic conditions to modify -X enables to form a derivative. The fluorine-containing pyrazole compound of one embodiment is also useful in the field of electronic materials such as organic semiconductors and liquid crystals.

R is not particularly limited as long as it is a hydrocarbon group having 1 to 12 carbon atoms and is composed of a carbon atom and a hydrogen atom, and includes a chain hydrocarbon group, an aromatic hydrocarbon group, an alicyclic hydrocarbon group and the like. The chain hydrocarbon group is not particularly limited as long as the total number of carbon atoms is 1 to 12 and may be a branched chain hydrocarbon group or a chain hydrocarbon group having no branch. The aromatic hydrocarbon group is not particularly limited as long as the total number of carbon atoms is 5 to 12 and may even be an aromatic hydrocarbon group having a substituent or an aromatic hydrocarbon group having no substituent. Moreover, the aromatic hydrocarbon group may have a condensed polycyclic structure. The alicyclic hydrocarbon group is not particularly limited as long as the total number of carbon atoms is 3 to 12 and may even be an alicyclic hydrocarbon group having a substituent or an alicyclic hydrocarbon group having no substituent. Further, the alicyclic hydrocarbon group may have a bridged ring structure.

Examples of the chain hydrocarbon group include alkyl groups such as a methyl group, an ethyl group, an n-propyl group, an i-propyl group, a n-butyl group, an i-butyl group, a sec-butyl group, a t-butyl group, a pentyl group, a hexyl group, a heptyl group, an octyl group, a nonyl group, a decyl group, an undecyl group, a dodecyl group;
alkenyl groups such as an ethenyl group, a propenyl group, a butenyl group, a pentenyl group, a hexenyl group, a heptenyl group, an octenyl group, a nonenyl group, a decenyl group, an undecenyl group, a dodecenyl group; and
alkynyl groups such as an ethynyl group, a propynyl group, a butynyl group, a pentynyl group, a hexynyl group, a heptynyl group, an octynyl group, a nonynyl group, a decynyl group, an undecynyl group, a dodecynyl group.

Examples of the aromatic hydrocarbon group include a phenyl group and a naphthyl group.

Examples of the alicyclic hydrocarbon group include a saturated or unsaturated cyclic hydrocarbon group, and examples of the cyclic hydrocarbon group include a cyclopropyl group, a cyclobutyl group, a cyclohexyl group, a cyclopentyl group, an adamantyl group, a norbornyl group and the like.

R is preferably an alkyl group having 1 to 10 carbon atoms. R being an alkyl group having 1 to 10 carbon atoms can easily prepare the fluoroisobutylene derivative of the general formula (2) and the fluoroisobutane derivative of the general formula (6), which are raw materials of the fluorine-containing pyrazole compound.

A group X represents a heterocyclic group containing at least one heteroatom selected from the group consisting of a halogen atom, nitrogen and oxygen as a ring atom, -OA¹, or -NA¹A², and A¹ and A² each independently represent a hydrogen atom or an organic group having 1 to 10 carbon atoms. When the group X is a halogen atom, examples thereof can include a fluorine atom (F), a chlorine atom (Cl), a bromine atom (Br), and an iodine atom (I), with the fluorine atom being preferred. When the group X is a heterocyclic group, the heterocyclic group contains nitrogen, oxygen, or both nitrogen and oxygen as ring atoms. Moreover, the number of ring atoms for at least one heteroatom selected from the group consisting of nitrogen and oxygen is not particularly limited as long as it is one or more, and may be one, or two or more, and includes, for example, one, two, three or four as the number of ring atoms for the heteroatom. The number of ring atoms constituting the heterocyclic group that is the group X is not particularly limited, and the number of ring atoms is preferably 3 to 14, more preferably 4 to 10, and still more preferably 5 to 6. The heterocyclic group that is the group X, may have a monocyclic structure or a multicyclic structure, and may also be an aromatic heterocyclic group or an alicyclic heterocyclic group. The heterocyclic group that is the group X, may or may not further have a substituent bonded to the ring atom, and can impart desired properties to the fluorine-containing pyrazole compound depending on the number and type of the heteroatoms, a size of the ring, the number of ring atoms, and the number, type, and presence or absence of substituents or the like.

More specifically, examples of the heterocyclic group that is the group X include a pyrrolyl group, a pyridyl group, an oxazolyl group, an isooxazolyl group, an imidazolyl group, a pyrazolyl group, a pyrazinyl group, a pyrimidinyl group, a pyridazinyl group, a benzofuranyl group, an isobenzofuranyl group, an indolyl group, an isoindolyl group, a quinolinyl group, an isoquinolinyl group, a benzimidazolyl group, a quinoxalinyl group, a quinazolinyl group, a cinnolinyl group, a phthalazinyl group, a carbazolyl group, a xanthenyl group, a phenanthridinyl group, a phenoxazinyl group, a pyrrolidinyl group, an imidazolidinyl group, a pyrazolidinyl group, a piperidinyl group, a piperazinyl group, a morpholinyl group, an indolinyl group, an isoindolinyl group, an azonanyl group, a thiazolyl group, a phenothiazinyl group, and the like. A substituent may or may not be further bonded to the ring atom in the heterocyclic group that is the group X. Examples of the substituent bonded to the ring atom include a halogen atom, a hydrocarbon group, an oxygen-containing substituent, a nitrogen-containing substituent, and sulfur-containing substituent.

A¹ included in -OA¹ that is the group X, represents a hydrogen atom or an organic group having 1 to 10 carbon atoms. Moreover, A¹ and A² included in -NA¹A² that is the group X each independently represent a hydrogen atom or an organic group having 1 to 10 carbon atoms. When A¹ and A² represent an organic group having 1 to 10 carbon atoms, A¹ and A² may have an atom other than a carbon atom and a hydrogen atom, and A¹ and A² can be, for example, hydrocarbon groups having 1 to 10 carbon atoms, heterocyclic groups, or oxygen-containing substituents in R above, and examples of the hydrocarbon group having 1 to 10 carbon atoms include an aromatic hydrocarbon group, an alkyl group, and the like. More specifically, examples of A¹ and A² include an oxanyl group, a phenyl group having or not having a substituent, a phenylalkyl group having or not having a substituent, an alkoxyalkyl group, a hydroxyalkyl group, a thiazolyl group, and the like.

Examples of the fluorine-containing pyrazole compound represented by the general formula (1) specifically include the following compounds.

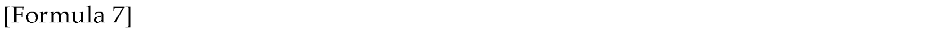

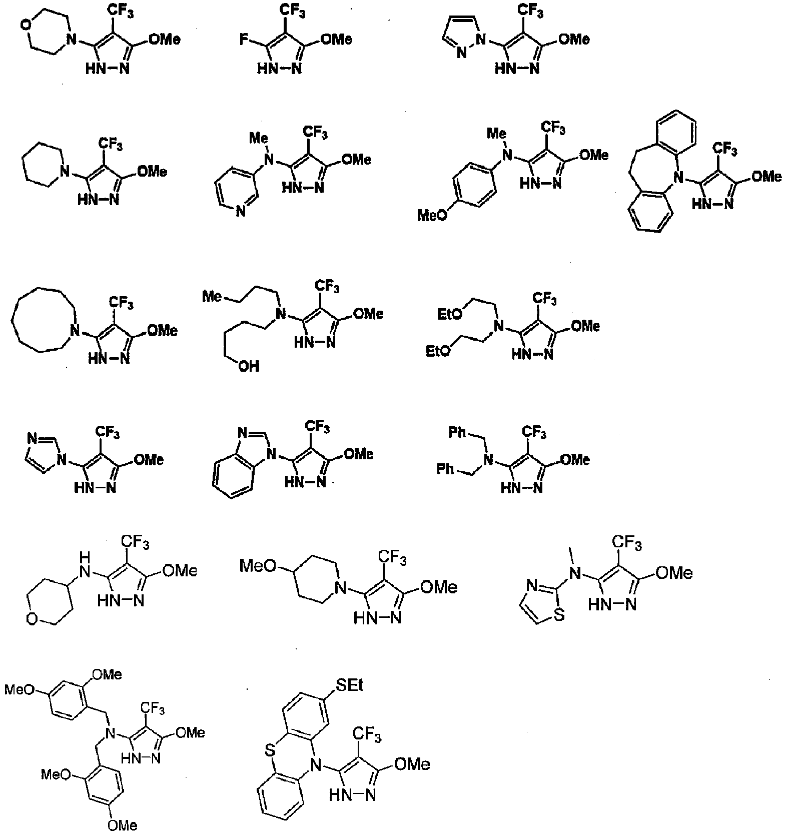

### (Method for producing fluorine-containing pyrazole compound)

The method for producing a fluorine-containing pyrazole compound according to one embodiment includes
(a) reacting a fluoroisobutylene derivative represented by the following general formula (2) with a compound represented by the following general formula (3) or a salt thereof to obtain a fluorine-containing pyrazole compound represented by the following general formula (4): 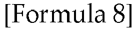 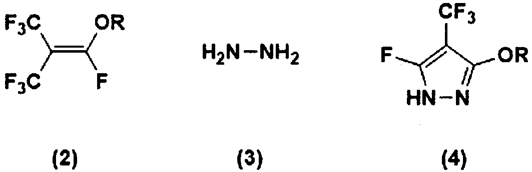 wherein in formulae (2) to (4) above, R represents a hydrocarbon group having 1 to 12 carbon atoms.

Preferably, the above (a) obtaining the fluorine-containing pyrazole compound is preferably carried out in the presence of a fluoride ion scavenger. The fluoroisobutylene derivative represented by the general formula (2) is preferably reacted with the compound represented by the general formula (3) or a salt thereof in the presence of the fluoride ion scavenger. The fluoride ion scavenger is not particularly limited as long as it is a substance having a function of capturing fluorine ions, and examples of the fluoride ion scavenger include lithium, sodium, magnesium, potassium, calcium, tetramethylammonium, trifluoroacetic acid, heptafluorobutyric acid, methanesulfonic acid, ethanesulfonic acid, benzenesulfonic acid, toluenesulfonic acid, trifluoromethanesulfonic acid, nonafluorobutanesulfonic acid, bis(trifluoromethanesulfonyl)imide, bis(nonafluorobutanesulfonyl)imide, N,N-hexafluoropropane-1,3-disulfonylimide, tetraphenylboric acid, tetrakis[3,5-bis(trifluoromethyl)phenyl]boric acid, tetrakis(pentafluorophenyl)borate. It is conceivable that a cation derived from the fluoride ion scavenger captures fluorine ions free from the fluoroisobutylene derivative represented by the general formula (2) during reaction, allowing a salt having low solubility to precipitate in an organic solvent to promote a reaction, and enabling to obtain the fluorine-containing pyrazole compound represented by the general formula (4) above in a high yield. R in the general formulae (2) and (4) above preferably represents an alkyl group having 1 to 10 carbon atoms.

A reaction of (a) above between the fluoroisobutylene derivative represented by the general formula (2) and the compound represented by the general formula (3) is represented by the following reaction formula (A).

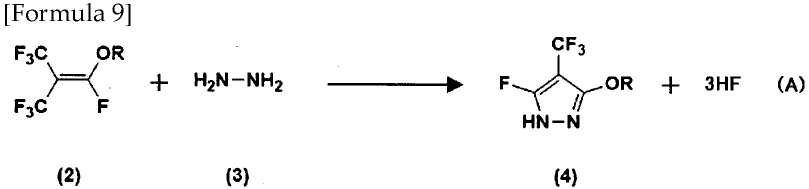

In the reaction formula (A) above, the compound of the general formula (3) may be in a form of salt. The form of salt can include a form of the amino moiety (-NH₂) constituting the amidino group of the compound of the general formula (3), being cationized to (-NH₃⁺) to form a salt with the counterion. The counterion is not particularly limited as long as it is a monovalent anion, and includes, for example, halide ions such as F, CF, Br, and I.

In the method for producing the fluorine-containing pyrazole compound according to one embodiment, reaction (A) above can be carried out in one step. Therefore, the fluorine-containing pyrazole compound of the general formula (4) above can be easily obtained. Incidentally, the reaction of (a) above forms a cyclic pyrazole structure between the fluoroisobutylene derivative of the general formula (2) and the amidino group of the compound of the general formula (3). F, CF₃, and -OR derived from the fluoroisobutylene derivative are located at the 3-position, 4-position, and 5-position of the pyrazole structure, respectively.

The reaction of (a) above is preferably carried out in the presence of the hydrogen halide scavenger. The hydrogen halide scavenger is a substance having a function of capturing hydrogen fluoride (HF) formed from a hydrogen atom derived from the amidino group in the compound of the general formula (3) and a fluorine atom derived from the fluoroisobutylene derivative of the general formula (2) in reaction formula (A) above. The hydrogen halide scavenger that is sodium hydroxide, potassium hydroxide, calcium hydroxide, sodium carbonate, potassium carbonate, sodium fluoride and potassium fluoride, and organic nitrogen derivatives such as pyridine, triethylamine, diisopropylethylamine, diazabicyclo nonane and diazabicyclo undecene, methyl triazabicyclodecene, diazabicyclo octane, and phosphazene base, can be used.

A reaction temperature upon reaction of (a) above is preferably 0 to 100°C, more preferably 5 to 80°C, and still more preferably 10 to 40°C. A reaction time upon reaction of (a) above is preferably 1 to 36 hours, more preferably 2 to 24 hours, and still more preferably 4 to 18 hours.

A solvent used in reaction (a) above includes aprotic polar solvents such as tetrahydrofuran, monoglyme, diglyme, triglyme, tetraglyme, acetonitrile, dimethylformamide, dimethylacetamide, methylpyrrolidone, dimethylethyleneurea, tetramethylurea, dimethylsulfoxide and sulfolane, or two-phase solvents of a protonic polar solvent such as water, and a water-insoluble solvent such as dichloromethane, toluene and diethyl ether. Moreover, as a catalyst for reaction of (a) above, quaternary ammonium halides such as benzyltriethylammonium chloride, a quaternary phosphonium halide, and crown ether, can be used.

The method for producing a fluorine-containing pyrazole compound of another embodiment includes
(b) reacting a fluoroisobutylene derivative represented by the following general formula (2) with a compound represented by the following general formula (3) or a salt thereof and a compound represented by the following general formula (5) to obtain a fluorine-containing pyrazole compound represented by the following general formula (1): 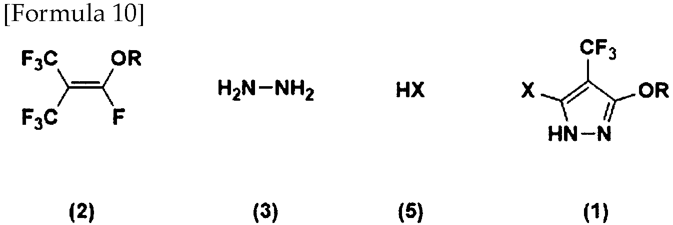
wherein in formulae (1) to (3) and (5) above, R represents a hydrocarbon group having 1 to 12 carbon atoms,
X represents a heterocyclic group containing at least one heteroatom selected from the group consisting of a halogen atom, nitrogen and oxygen as a ring atom, -OA¹, or -NA¹A², and
A¹ and A² each independently represent a hydrogen atom or an organic group having 1 to 10 carbon atoms.

Preferably, step (b) above of obtaining the fluorine-containing pyrazole compound is preferably carried out in the presence of a fluoride ion scavenger. The fluoroisobutylene derivative represented by the general formula (2) above is preferably reacted with the compound represented by the general formula (3) above or a salt thereof and the compound represented by the general formula (5) above in the presence of a fluoride ion scavenger. The fluoride ion scavenger is not particularly limited as long as it is a substance having a function of capturing fluorine ions, and examples thereof include the same fluoride ion scavenger as the fluoride ion scavenger used in step (a) above. It is conceivable that a cation derived from the fluoride ion scavenger captures a fluorine ion free from the fluoroisobutylene derivative represented by the general formula (2) during reaction, allowing a salt having low solubility to precipitate in an organic solvent to promote a reaction, and enabling to obtain the fluorine-containing pyrazole compound represented by the general formula (1) above in a high yield. R in the general formulae (1) and (2) above preferably represents an alkyl group having 1 to 10 carbon atoms.

The reaction of (b) above between the fluoroisobutylene derivative represented by the general formula (2) and the compounds represented by the general formulae (3) and (5) is represented by the following reaction formula (B).

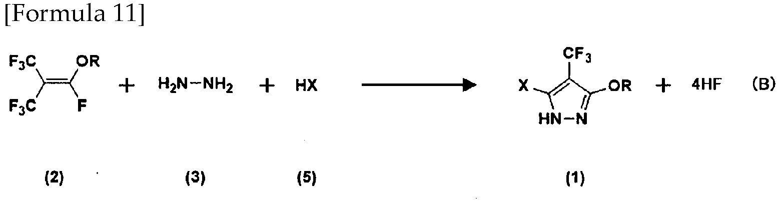

In the aforementioned reaction formula (B), the compound of the general formula (3) may be in a form of salt. The form of salt can include a form of the amino moiety (-NH₂) constituting the amidino group of the compound of the general formula (3), being cationized to (-NH₃⁺) to form a salt with the counterion. The counterion is not particularly limited as long as it is a monovalent anion, and includes, for example, halide ions such as F⁻, Cl⁻, Br, and I.

When the group X is a halogen atom, examples thereof can include a fluorine atom (F), a chlorine atom (Cl), a bromine atom (Br), and an iodine atom (I), with the fluorine atom being preferred. When the group X is a heterocyclic group, the heterocyclic group contains nitrogen, oxygen, or both nitrogen and oxygen as ring atoms. Moreover, the number of ring atoms for at least one heteroatom selected from the group consisting of nitrogen and oxygen is not particularly limited as long as it is one or more, and may be one, or two or more, and includes, for example, one, two, three or four as the number of ring atoms for the heteroatom. The number of ring atoms constituting the heterocyclic group that is the group X is not particularly limited, and the number of ring atoms is preferably 3 to 14, more preferably 4 to 10, and still more preferably 5 to 6. The heterocyclic group that is the group X may have a monocyclic structure or a multicyclic structure, and may also be an aromatic heterocyclic group or an alicyclic heterocyclic group. A¹ included in -OA¹ that is the group X, represents a hydrogen atom or an organic group having 1 to 10 carbon atoms. Moreover, A¹ and A² included in -NA¹A² that is the group X each independently represent a hydrogen atom or an organic group having 1 to 10 carbon atoms. When A¹ and A² represent an organic group having 1 to 10 carbon atoms, A¹ and A² may have an atom other than a carbon atom and a hydrogen atom, and A¹ and A² can be, for example, hydrocarbon groups, heterocyclic groups, or oxygen-containing substituents, which have 1 to 10 carbon atoms in R above, and examples of the hydrocarbon group having 1 to 10 carbon atoms include an aromatic hydrocarbon group, an alkyl group, and the like.

In the method for producing the fluorine-containing pyrazole compound according to another embodiment, the reaction of (B) above can be carried out in one step. Therefore, the fluorine-containing pyrazole compound of the general formula (1) above can be easily obtained. Incidentally, the reaction of (b) above forms a cyclic pyrazole structure between the fluoroisobutylene derivative of the general formula (2) and the amidino group of the compound of the general formula (3). X derived from the compound represented by the general formula (5), and CF₃ and -OR which are derived from the fluoroisobutylene derivative, are located at the 3-position, 4-position, and 5-position of the pyrazole structure, respectively.

A reaction temperature upon reaction of (b) above is preferably 0 to 100°C, more preferably 5 to 80°C, and still more preferably 10 to 40°C. A reaction time upon reaction of (b) above is preferably 1 to 36 hours, more preferably 2 to 24 hours, and still more preferably 4 to 18 hours. In the reaction of (b) above, the same hydrogen halide scavenger and solvent as in (a) above can be used.

The method for producing a fluorine-containing pyrazole compound of another embodiment includes
(c) reacting a fluoroisobutane derivative represented by the following general formula (6) with a compound represented by the following general formula (3) or a salt thereof to obtain a fluorine-containing pyrazole compound represented by the following general formula (4): 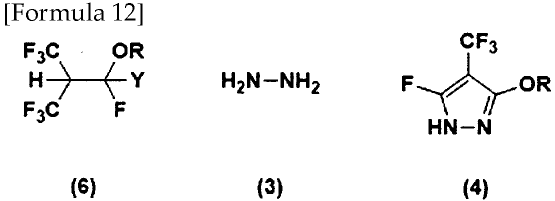
wherein in formulae (3), (4), and (6) above, R represents a hydrocarbon group having 1 to 12 carbon atoms,
Y represents a halogen atom, -OA³, -SOₘA³ where m is an integer of 0 to 3, or - NA³A⁴, and
A³ and A⁴ each independently represent a hydrogen atom or a hydrocarbon group having 1 to 10 carbon atoms.

Preferably, step (c) above of obtaining the fluorine-containing pyrazole compound is preferably carried out in the presence of a fluoride ion scavenger. The fluoroisobutane derivative represented by the general formula (6) above is preferably reacted with the compound represented by the general formula (3) above or a salt thereof in the presence of a fluoride ion scavenger. The fluoride ion scavenger is not particularly limited as long as it is a substance having a function of capturing fluorine ions, and examples of the fluoride ion scavenger include lithium, sodium, magnesium, potassium, calcium, tetramethylammonium, trifluoroacetic acid, and heptafluorobutyric acid, methanesulfonic acid, ethanesulfonic acid, benzenesulfonic acid, toluenesulfonic acid, trifluoromethanesulfonic acid, nonafluorobutanesulfonic acid, bis(trifluoromethanesulfonyl)imide, bis(nonafluorobutanesulfonyl)imide, N,N-hexafluoropropane-1,3-disulfonylimide, tetraphenylboric acid, tetrakis[3,5-bis(trifluoromethyl)phenyl]boric acid, tetrakis(pentafluorophenyl)borate. It is conceivable that a cation derived from the fluoride ion scavenger captures a fluorine ion free from the fluoroisobutane derivative represented by the general formula (6) during reaction, allowing a salt having low solubility to precipitate in an organic solvent to promote a reaction, and enabling to obtain the fluorine-containing pyrazole compound represented by the general formula (4) in a high yield. R in the compounds of the general formulae (4) and (6) in step (c) above preferably represents an alkyl group having 1 to 10 carbon atoms.

The reaction of (c) above between the fluoroisobutane derivative represented by the general formula (6) and the compound represented by the general formula (3) is represented by the following reaction formula (C).

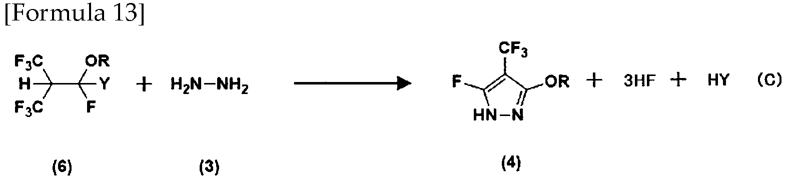

In the aforementioned reaction formula (C), the compounds of the general formula (3) each may be in a form of salt. The form of salt can include a form of the amino moiety (-NH₂) constituting the amidino group of the compound of the general formula (3), being cationized to (-NH₃⁺) to form a salt with the counterion. The counterion is not particularly limited as long as it is a monovalent anion, and includes, for example, halide ions such as F, CF, Br, and I.

Examples of the halogen atom that is Y can include F, Cl, Br, and I. A³ included in -OA³ and -SOₘA³ that are Y where m is an integer of 0 to 3 represents a hydrogen atom or a hydrocarbon group having 1 to 10 carbon atoms. A³ and A⁴ included in -NA³A⁴ that is Y, each independently represent a hydrogen atom or a hydrocarbon group having 1 to 10 carbon atoms. When A³ and A⁴ represent a hydrocarbon group having 1 to 10 carbon atoms, they can be, for example, hydrocarbon groups having 1 to 10 carbon atoms in R above.

In the method for producing the fluorine-containing pyrazole compound according to another embodiment, the reaction of (C) above can be carried out in one step. Therefore, the fluorine-containing pyrazole compound of the general formula (4) above can be easily obtained. Incidentally, the reaction of (c) above forms a cyclic pyrazole structure between the fluoroisobutane derivative of the general formula (6) and the amidino group of the compound of the general formula (3). F, CF₃, and -OR derived from the fluoroisobutane derivative are located at the 3-position, 4-position, and 5-position of the pyrazole structure, respectively.

A reaction temperature upon reaction of (c) above is preferably 0 to 100°C, more preferably 5 to 80°C, and still more preferably 10 to 40°C. A reaction time upon reaction of (c) above is preferably 1 to 36 hours, more preferably 2 to 24 hours, and still more preferably 4 to 18 hours. In the reaction of (c) above, the same hydrogen halide scavenger and solvent as in (a) above can be used.

The method for producing a fluorine-containing pyrazole compound of the other embodiment includes
(d) reacting a fluoroisobutane derivative represented by the following general formula (6) with a compound represented by the following general formula (3) or a salt thereof and a compound represented by the following general formula (5) to obtain a fluorine-containing pyrazole compound represented by the following general formula (1): 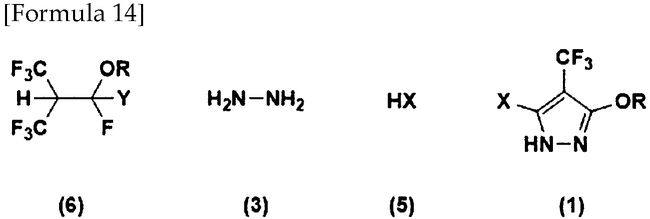
wherein in formulae (1), (3), (5), and (6) above, R represents a hydrocarbon group having 1 to 12 carbon atoms,
X represents a heterocyclic group containing at least one heteroatom selected from the group consisting of a halogen atom, nitrogen and oxygen as a ring atom, -OA¹, or -NA¹A²,
A¹ and A² each independently represent a hydrogen atom or an organic group having 1 to 10 carbon atoms,
Y represents a halogen atom, -OA³, -SOₘA³ where m is an integer of 0 to 3, or - NA³A⁴, and
A³ and A⁴ each independently represent a hydrogen atom or a hydrocarbon group having 1 to 10 carbon atoms.

Preferably, step (d) above of obtaining the fluorine-containing pyrazole compound is preferably carried out in the presence of a fluoride ion scavenger. The fluoroisobutane derivative represented by the general formula (6) above is preferably reacted with the compound represented by the general formula (3) above or a salt thereof and the compound represented by the general formula (5) above in the presence of a fluoride ion scavenger. The fluoride ion scavenger is not particularly limited as long as it is a substance having a function of capturing fluorine ions, and examples thereof can include the same fluoride ion scavenger as the fluoride ion scavenger used in (c) above. It is conceivable that a cation derived from the fluoride ion scavenger captures a fluorine ion free from the fluoroisobutane derivative represented by the general formula (6) during reaction, allowing a salt having low solubility to precipitate in an organic solvent to promote a reaction, and enabling to obtain the fluorine-containing pyrazole compound represented by the general formula (1) above in a high yield. R in the compounds of the general formulae (1) and (6) in step (d) above preferably represents an alkyl group having 1 to 10 carbon atoms.

The reaction of (d) above between the fluoroisobutane derivative represented by the general formula (6) and the compounds represented by the general formulae (3) and (5) is represented by the following reaction formula (D).

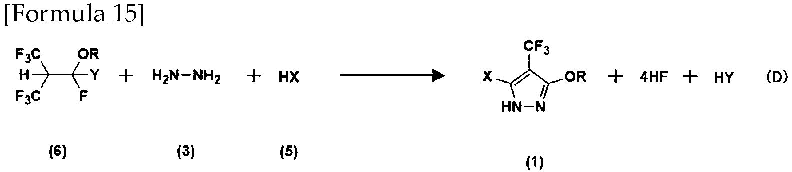

In the aforementioned reaction formula (D), the compounds of the general formula (3) each may be in a form of salt. The form of salt can include a form of the amino moiety (-NH₂) constituting the amidino group of the compound of the general formula (3), being cationized to (-NH₃⁺) to form a salt with the counterion. The counterion is not particularly limited as long as it is a monovalent anion, and includes, for example, halide ions such as F, CF, Br, and I.

When the group X is a halogen atom, examples thereof include a fluorine atom (F), a chlorine atom (Cl), a bromine atom (Br), and an iodine atom (I), with the fluorine atom being preferred. When the group X is a heterocyclic group, the heterocyclic group contains nitrogen, oxygen, or both nitrogen and oxygen as ring atoms. Further, the number of ring atoms for at least one heteroatom selected from the group consisting of nitrogen and oxygen is not particularly limited as long as it is one or more, and may be one, or two or more, and for example, examples of the number of ring atoms for the heteroatom include one, two, three, or four. The number of ring atoms constituting the heterocyclic group that is the group X is not particularly limited, and the number of ring atoms is preferably 3 to 14, more preferably 4 to 10, and still more preferably 5 to 6. The heterocyclic group that is the group X may have a monocyclic structure or a multicyclic structure, and may also be an aromatic heterocyclic group or an alicyclic heterocyclic group. A¹ included in -OA¹ that is the group X, represents a hydrogen atom or an organic group having 1 to 10 carbon atoms. Moreover, A¹ and A² included in -NA¹A² that is the group X, each independently represent a hydrogen atom or an organic group having 1 to 10 carbon atoms. When A¹ and A² represent an organic group having 1 to 10 carbon atoms, A¹ and A² may have an atom other than a carbon atom and a hydrogen atom, and A¹ and A² can be, for example, hydrocarbon groups, heterocyclic groups, or oxygen-containing substituents, which have 1 to 10 carbon atoms in R above, and examples of the hydrocarbon group having 1 to 10 carbon atoms include an aromatic hydrocarbon group, an alkyl group, and the like.

Examples of the halogen atom that is Y include F, Cl, Br, and I. A³ included in -OA³ and -SOₘA³ that are Y where m is an integer of 0 to 3 represents a hydrogen atom or a hydrocarbon group having 1 to 10 carbon atoms. A³ and A⁴ included in -NA³A⁴ that is Y, each independently represent a hydrogen atom or a hydrocarbon group having 1 to 10 carbon atoms. When A³ and A⁴ represent a hydrocarbon group having 1 to 10 carbon atoms, they can be, for example, hydrocarbon groups having 1 to 10 carbon atoms in R above.

In the method for producing the fluorine-containing pyrazole compound according to the other embodiment, the reaction of (D) above can be carried out in one step. Therefore, the fluorine-containing pyrazole compound of the general formula (1) above can be easily obtained. Incidentally, the reaction of (d) above forms a cyclic pyrazole structure between the fluoroisobutane derivative of the general formula (6) and the amidino group of the compound of the general formula (3). X derived from the compound represented by the general formula (5), and CF₃ and -OR which are derived from the fluoroisobutane derivative, are located, at the 3-position, 4-position, and 5-position of the pyrazole structure, respectively.

A reaction temperature upon reaction of (d) above is preferably 0 to 100°C, more preferably 5 to 80°C, and still more preferably 10 to 40°C. A reaction time upon reaction of (d) above is preferably 1 to 36 hours, more preferably 2 to 24 hours, and still more preferably 4 to 18 hours. In the reaction of (d) above, the same hydrogen halide scavenger as in (a) above can be used.

In the reaction of (d) above, the same solvent and catalyst as in the reaction of (c) above can be used.

Although the embodiments of the present invention have been described above, the present invention is not limited to the aforementioned embodiments, and encompasses all aspects included in the concept and claims of the present invention and can be modified within the scope of the present invention in various embodiments.

### Examples

Next, in order to further clarify the effect of the present invention, Examples will be described, but the present invention is not limited to these Examples.

### (Example 1)

### Production of 5-methoxy-3-(4-morpholyl)-4-trifluoromethylpyrazole

Under ice-water cooling, 2.0 g (19 mmol) of hydrazine dihydrochloride and 4.0 g (19 mmol) of 1,3,3,3-tetrafluoro-1-methoxy-2-trifluoromethyl-1-propene were added to 30 g of MeOH to obtain a solution 1. Subsequently, a solution 2 in which 5.4 g (42 mmol) of diisopropylethylamine was dissolved in 15 g of MeOH was added dropwise to the solution 1 such that the internal temperature did not exceed 10°C, and the resultant mixed solution was raised to room temperature. After about 16 hours, the mixed solution was cooled with ice water, to the mixed solution was added dropwise a solution 3 in which 9.1 g (105 mmol) of morpholine was dissolved in 20 g of MeOH, and was raised to room temperature. After about 8 hours, the reaction mixture was purified on a silica gel column using a mixed solvent of hexane-ethyl acetate = 7: 3 to isolate 1.2 g of 5-methoxy-3-(4-morpholyl)-4-trifluoromethylpyrazole represented by the following formula (E). The isolated yield of 5-methoxy-3-(4-morpholyl)-4-trifluoromethylpyrazole was 25%.

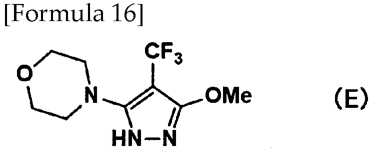

The analysis results of the target product obtained are as follows.
Mass Spectrum (APCl, m/z): 251 ([M]⁺)
¹H-NMR (400 MHz, CDCl₃) δ ppm: 3.91 (s, 3H), 3.81 (dd, 4H), 3.14 (dd, 4H)

### (Example 2)

### Production of 3-fluoro-5-methoxy-4-trifluoromethylpyrazole

Under ice-water cooling, 2.0 g (19 mmol) of hydrazine dihydrochloride and 4.0 g (19 mmol) of 1,3,3,3-tetrafluoro-1-methoxy-2-trifluoromethyl-1-propene were added to 30 g of THF to obtain a solution 1. Subsequently, a solution 2 in which 14 g (105 mmol) of diisopropylethylamine was dissolved in 30 g of THF was added dropwise to the solution 1 such that the internal temperature did not exceed 10°C, and the resultant was raised to room temperature. After about 16 hours, the reaction mixture was purified on a silica gel column using a mixed solvent of hexane-ethyl acetate = 7: 3 to isolate 70 mg of 3-fluoro-5-methoxy-4-trifluoromethylpyrazole represented by the following formula (F). The isolated yield of 3-fluoro-5-methoxy-4-trifluoromethylpyrazole was 2%.

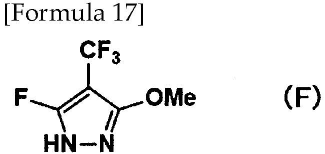

The analysis results of the target product obtained are as follows.
Mass Spectrum (APCl, m/z): 184 ([M]⁺)

### (Example 3)

### Production of 5-methoxy-3-(4-morpholyl)-4-trifluoromethylpyrazole by using 1,1,1,3,3-pentafluoro-3-methoxy-2-trifluoromethyl-propane instead of 1,3,3,3-tetrafluoro-1-methoxy-2-trifluoromethyl-1-propene in Example 1

Under ice-water cooling, 2.0 g (19 mmol) of hydrazine dihydrochloride and 4.4 g (19 mmol) of 1,1,1,3,3-pentafluoro-3-methoxy-2-trifluoromethyl-propane were added to 30 g of MeOH to obtain a solution 1. Subsequently, a solution 2 in which 8.1 g (63 mmol) of diisopropylethylamine was dissolved in 20 g of MeOH was added dropwise to the solution 1 such that the internal temperature did not exceed 10°C, and the resultant mixed solution was raised to room temperature. After about 16 hours, the mixed solution was cooled with ice water, to the mixed solution was added dropwise a solution 3 in which 9.1 g (105 mmol) of morpholine was dissolved in 20 g of MeOH, followed by raising to room temperature. After about 8 hours, the reaction mixture was purified on a silica gel column using a mixed solvent of hexane-ethyl acetate = 7: 3 to isolate 5-methoxy-3-(4-morpholyl)-4-trifluoromethylpyrazole. The analysis results of the target product obtained were the same as those of the product of Example 1.

### (Example 4)

### Production of 5-methoxy-3-(1-pyrazolyl)-4-trifluoromethylpyrazole

Under ice-water cooling, 2.0 g (19 mmol) of hydrazine dihydrochloride and 4.0 g (19 mmol) of 1,3,3,3-tetrafluoro-1-methoxy-2-trifluoromethyl-1-propene were added to 30 g of MeOH to obtain a solution 1. Subsequently, a solution 2 in which 3.8 g (38 mmol) of triethylamine was dissolved in 15 g of MeOH was added dropwise to the solution 1 such that the internal temperature did not exceed 10°C, and the resultant mixed solution was raised to room temperature. After about 16 hours, the mixed solution was cooled with ice water, to the mixed solution was added dropwise a solution 3 in which 1.3 g (19 mmol) of pyrazole and 8.5 g (84 mmol) of triethylamine were dissolved in 30 g of MeOH, followed by raising to room temperature. After about 8 hours, the reaction mixture was purified on a silica gel column using a mixed solvent of hexane-ethyl acetate = 7: 3 to isolate 0.2 g of 5-methoxy-3-(1-pyrazolyl)-4-trifluoromethylpyrazole represented by the following formula (G). The isolated yield of 5-methoxy-3-(1-pyrazolyl)-4-trifluoromethylpyrazole was 5%.

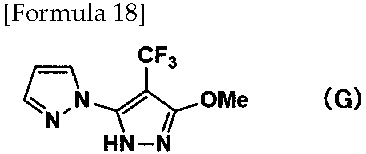

The analysis results of the target product obtained are as follows.
Mass Spectrum (APCl, m/z): 232 ([M]⁺)
¹H-NMR (400 MHz, CDCl₃) δ ppm: 7.90 (d, 1H), 7.78 (d, 1H), 6.52 (dd, 1H), 4.03 (s, 3H)

### (Example 5)

### Production of 1-(3-methoxy-4-trifluoromethyl-1H-pyrazol-5-yl)azonane

0.5 g (4.8 mmol) of hydrazine dihydrochloride was dissolved in 13 ml of methanol, and the solution was cooled to 0°C, to the solution were added 1.0 g (4.7 mmol) of 1,3,3,3-tetrafluoro-1-methoxy-2-trifluoromethyl-1-propene and 1.0 g (9.6 mmol) of triethylamine such that the internal temperature did not exceed room temperature, followed by raising to room temperature. The solution obtained was stirred for 16 hours, then cooled to 0°C, to the solution was added 3.1 g (24.0 mmol) of octamethyleneimine such that the internal temperature did not exceed room temperature, followed by raising to room temperature. Next, after the solution was stirred for 8 hours, the reaction mixture obtained was purified on a silica gel column using a mixed solvent of hexane-ethyl acetate to isolate 0.6 g (1.9 mmol) of 1-(3-methoxy-4-trifluoromethyl-1H-pyrazol-5-yl)azonane represented by the following formula (H). The isolated yield of 1-(3-methoxy-4-trifluoromethyl-1H-pyrazol-5-yl)azonane was 41%.

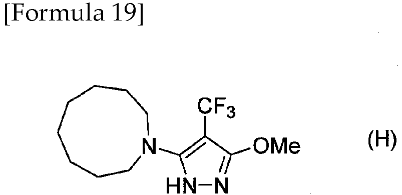

The analysis results of the target product obtained are as follows.
Mass Spectrum (APCl, m/z): 292.6 ([M+H]⁺)
¹H-NMR (400 MHz, CDCl₃) δ ppm: 3.90 (s, 3H), 3.25-3.32 (m, 4H), 1.62-1.72 (m, 8H), 1.49-1.59 (m, 4H)

### (Example 6)

### Production of 3-methoxy-N-(oxan-4-yl)-4-trifluoromethyl-1H-pyrazol-5-amine

0.5 g (4.8 mmol) of hydrazine dihydrochloride was dissolved in 13 ml of methanol, and the solution was cooled to 0°C, then to the solution were added 1.0 (4.7 mmol) of 1,3,3,3-tetrafluoro-1-methoxy-2-trifluoromethyl-1-propene and 1.0 g (9.7 mmol) of triethylamine such that the internal temperature did not exceed room temperature, followed by raising to room temperature. After stirring for 16 hours, the solution obtained was cooled to 0°C, to the solution were added 0.7 g (4.8 mmol) of 4-aminotetrahydropyran hydrochloride and 2.4 g (23.9 mmol) of triethylamine such that the internal temperature did not exceed room temperature, followed by raising to room temperature. Next, after the solution was stirred for 8 hours, the reaction mixture obtained was purified on a silica gel column using a mixed solvent of hexane-ethyl acetate to isolate 0.1 g (0.5 mmol) of 3-methoxy-N-(oxan-4-yl)-4-trifluoromethyl-1H-pyrazol-5-amine represented by the following formula (I). The isolated yield of 3-methoxy-N-(oxan-4-yl)-4-trifluoromethyl-1H-pyrazol-5-amine was 10%.

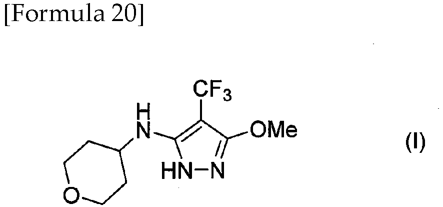

The analysis results of the target product obtained are as follows.
Mass Spectrum (APCl, m/z): 266.5 ([M+H]⁺)
¹H-NMR (400 MHz, CDCl₃) δ ppm: 4.11-4.16 (m, 1H), 4.00 (ddd, J = 11.9, 3.7, 3.3 Hz, 2H), 3.90 (s, 3H), 3.47 (ddd, J = 11.9, 11.3, 2.1 Hz, 2H), 3.28-3.39 (m, 1H), 1.95-2.02 (m, 2H), 1.51-1.62 (m, 2H)

### (Example 7)

### Production of N,N-bis[(2,4-dimethoxyphenyl)methyl]-3-methoxy-4-trifluoromethyl-1H-pyrazol-5-amine

0.5 g (4.8 mmol) of hydrazine dihydrochloride was dissolved in 13 ml of methanol, and the solution was cooled to 0°C, then to the solution were added 1.0 (4.7 mmol) of 1,3,3,3-tetrafluoro-1-methoxy-2-trifluoromethyl-1-propene and 1.0 g (9.6 mmol) of triethylamine such that the internal temperature did not exceed room temperature, followed by raising to room temperature. After stirring for 16 hours, the solution obtained was cooled to 0°C, to the solution were added 1.5 g (4.8 mmol) of bis(2,4-dimethoxybenzyl)amine and 1.9 g (19.1 mmol) of triethylamine such that the internal temperature did not exceed room temperature, followed by raising to room temperature. Next, after the solution was stirred for 8 hours, the reaction mixture obtained was purified on a silica gel column using a mixed solvent of hexane-ethyl acetate to isolate 0.3 g (0.6 mmol) of N,N-bis[(2,4-dimethoxyphenyl)methyl]-3-methoxy-4-trifluoromethyl-1H-pyrazol-5-amine represented by the following formula (J). The isolated yield of N,N-bis[(2,4-dimethoxyphenyl)methyl]-3-methoxy-4-trifluoromethyl-1H-pyrazol-5-amine was 13%.

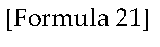

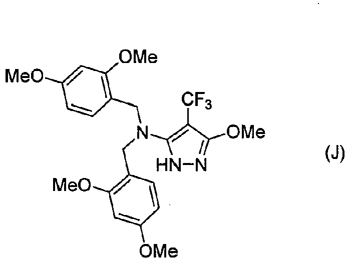

The analysis results of the target product obtained are as follows.
Mass Spectrum (APCl, m/z): 483.0 ([M+H]⁺)
¹H-NMR (400 MHz, CDCl₃) δ ppm: 9.19 (br, 1H), 7.15 (d, J = 8.3 Hz, 2H), 6.42-6.48 (m, 4H), 4.24 (s, 3H), 3.87 (s, 3H), 3.80 (s, 1H)

### (Example 8)

### Production of 3-methoxy-N-(4-methoxyphenyl)-N-methyl-4-trifluoromethyl-1H-pyrazol-5-amine

0.5 g (4.8 mmol) of hydrazine dihydrochloride was dissolved in 13 ml of methanol, and the solution was cooled to 0°C, then to the solution were added 1.0 (4.7 mmol) of 1,3,3,3-tetrafluoro-1-methoxy-2-trifluoromethyl-1-propene and 1.0 g (9.8 mmol) of triethylamine such that the internal temperature did not exceed room temperature, followed by raising to room temperature. After stirring for 16 hours, the solution obtained was cooled to 0°C, to the solution were added 0.7 g (4.8 mmol) of N-methyl-p-anisidine and 1.9 g (19.3 mmol) of triethylamine such that the internal temperature did not exceed room temperature, followed by raising to room temperature. Next, after the solution was stirred for 8 hours, the reaction mixture obtained was purified on a silica gel column using a mixed solvent of hexane-ethyl acetate to obtain 0.1 g of a mixture of 3-methoxy-N-(4-methoxyphenyl)-N-methyl-4-trifluoromethyl-1H-pyrazol-5-amine represented by the following formula (K).

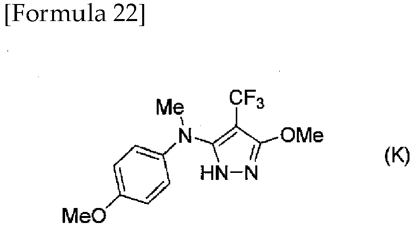

The analysis results of the target product obtained are as follows.
Mass Spectrum (APCl, m/z): 302.6 ([M+H]⁺)

### (Example 9)

### Production of 3-methoxy-5-(4-methoxypiperidin-1-yl)-4-trifluoromethyl-1H-pyrazole

0.5 g (4.8 mmol) of hydrazine dihydrochloride was dissolved in 13 ml of methanol, and the solution was cooled to 0°C, then to the solution were added 1.0 g (4.7 mmol) of 1,3,3,3-tetrafluoro-1-methoxy-2-trifluoromethyl-1-propene and 1.0 g (9.7 mmol) of triethylamine such that the internal temperature did not exceed room temperature, followed by raising to room temperature. After stirring for 17 hours, the solution obtained was cooled to 0°C, to the solution were added 0.6 g (4.8 mmol) of 4-methoxypiperidine and 1.9 g (19.2 mmol) of triethylamine such that the internal temperature did not exceed room temperature, followed by raising to room temperature. Next, after the solution was stirred for 8 hours, the reaction mixture obtained was purified on a silica gel column using a mixed solvent of hexane-ethyl acetate to obtain 0.2 g of a mixture of 3-methoxy-5-(4-methoxypiperidine-1-yl)-4-trifluoromethyl-1H-pyrazole represented by the following formula (L).

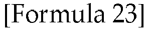

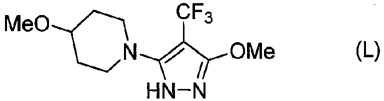

The analysis results of the target product obtained are as follows.
Mass Spectrum (APCl, m/z): 280.8 ([M+H]⁺)
¹⁹F-NMR (400 MHz, CDCl₃) δ ppm: -55.85 (s, 3F)

### (Example 10)

### Production of N,N-bis(2-ethoxyethyl)-3-methoxy-4-trifluoromethyl-1H-pyrazol-5-amine

0.5 g (4.8 mmol) of hydrazine dihydrochloride was dissolved in 13 ml of methanol, and the solution was cooled to 0°C, then to the solution were added 1.0 g (4.7 mmol) of 1,3,3,3-tetrafluoro-1-methoxy-2-trifluoromethyl-1-propene and 1.0 g (9.6 mmol) of triethylamine such that the internal temperature did not exceed room temperature, followed by raising to room temperature. After stirring for 16 hours, the solution obtained was cooled to 0°C, to the solution were added 0.8 g (4.9 mmol) of bis(2-ethoxyethyl)amine and 2.0 g (19.4 mmol) of triethylamine such that the internal temperature did not exceed room temperature, followed by raising to room temperature. Next, after the solution was stirred for 8 hours, the reaction mixture obtained was purified on a silica gel column using a mixed solvent of hexane-ethyl acetate to obtain 0.1 g of a mixture of N,N-bis(2-ethoxyethyl)-3-methoxy-4-trifluoromethyl-1H-pyrazol-5-amine represented by the following formula (M).

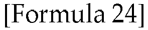

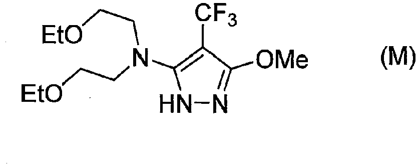

The analysis results of the target product obtained are as follows.
Mass Spectrum (APCl, m/z): 327.0 ([M+H]⁺)
¹H-NMR (400 MHz, CDCl₃) δ ppm: 10.38 (br, 1H), 3.90 (s, 1H), 3.61 (t, J = 4.7 Hz, 4H), 3.53 (q, J = 7.0 Hz, 4H), 3.46 (t, J = 4.9 Hz, 4H), 1.23 (t, J = 7.0 Hz, 6H)

### (Example 11)

### Production of 4-[butyl(3-methoxy-4-trifluoromethyl-1H-pyrazol-5-yl)amino]-1-butanol

0.5 g (4.8 mmol) of hydrazine dihydrochloride was dissolved in 13 ml of methanol, and the solution was cooled to 0°C, then to the solution were added 1.0 g (4.7 mmol) of 1,3,3,3-tetrafluoro-1-methoxy-2-trifluoromethyl-1-propene and 1.0 g (9.7 mmol) of triethylamine such that the internal temperature did not exceed room temperature, followed by raising to room temperature. After stirring for 16 hours, the solution obtained was cooled to 0°C, to the solution were added 0.7 g (5.0 mmol) of 4-(butylamino)-1-butanol and 1.9 g (19.2 mmol) of triethylamine such that the internal temperature did not exceed room temperature, followed by raising to room temperature. Next, after the solution was stirred for 8 hours, the reaction mixture obtained was purified on a silica gel column using a mixed solvent of hexane-ethyl acetate to obtain 0.1 g of a mixture of 4-[butyl(3-methoxy-4-trifluoromethyl-1H-pyrazol-5-yl)amino]-1-butanol represented by the following formula (N).

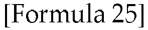

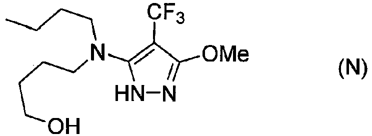

The analysis results of the target product obtained are as follows.
Mass Spectrum (APCl, m/z): 311.0 ([M+H]⁺)
¹⁹F-NMR (400 MHz, CDCl₃) δ ppm: -54.59 (s, 3F)

### (Example 12)

### Production of N-(3-methoxy-4-trifluoromethyl-1H-pyrazol-5-yl)-N-methyl-2-thiazolamine

0.5 g (4.8 mmol) of hydrazine dihydrochloride was dissolved in 13 ml of methanol, and the solution was cooled to 0°C, then to the solution were added 1.0 g (4.7 mmol) of 1,3,3,3-tetrafluoro-1-methoxy-2-trifluoromethyl-1-propene and 1.0 g (9.5 mmol) of triethylamine such that the internal temperature did not exceed room temperature, followed by raising to room temperature. After stirring for 17 hours, the solution obtained was cooled to 0°C, to the solution were added 0.5 g (4.7 mmol) of N-methyl-2-thiazolamine and 1.9 g (19.2 mmol) of triethylamine such that the internal temperature did not exceed room temperature, followed by raising temperature until reflux. Next, after stirring for 8 hours, the solution was air-cooled to room temperature, and the reaction mixture obtained was purified on a silica gel column using a mixed solvent of hexane-ethyl acetate to obtain 0.03 g of a mixture of N-(3-methoxy-4-trifluoromethyl-1H-pyrazol-5-yl)-N-methyl-2-thiazolamine represented by the following formula (O).

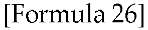

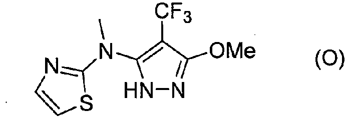

The analysis results of the target product obtained are as follows.
Mass Spectrum (APCl, m/z): 279.8 ([M+H]⁺)

### (Example 13)

### Production of 2-(ethylthio)-10-(3-methoxy-4-trifluoromethyl-1H-pyrazol-5-yl)-10H-phenothiazine

0.5 g (4.8 mmol) of hydrazine dihydrochloride was dissolved in 13 ml of methanol, and the solution was cool to 0°C, then to the solution were added 1.0 g (4.7 mmol) of 1,3,3,3-tetrafluoro-1-methoxy-2-trifluoromethyl-1-propene and 1.0 g (9.6 mmol) of triethylamine such that the internal temperature did not exceed room temperature, followed by raising to room temperature. After stirring for 16 hours, the solution obtained was cooled to 0°C, to the solution were added 1.2 g (4.8 mmol) of 2-ethylthiophenothiazine and 1.9 g (19.1 mmol) of triethylamine such that the internal temperature did not exceed room temperature, followed by raising to room temperature. Next, after the solution was stirred for 25 hours, the reaction mixture obtained was purified on a silica gel column using a mixed solvent of hexane-ethyl acetate to obtain 0.02 g of a mixture of 2-(ethylthio)-10-(3-methoxy-4-trifluoromethyl-1H-pyrazol-5-yl)-10H-phenothiazine represented by the following formula (P).

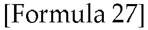

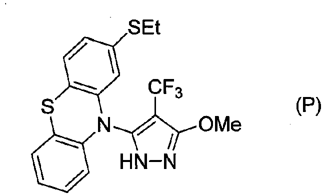

The analysis results of the target product obtained are as follows.
Mass Spectrum (EI, m/z): 423.1 ([M]⁺)

### Evaluation test for rice blast (500 ppm)

The fluorine-containing pyrazole compounds prepared in Examples 2 and 9 were each dissolved in DMSO (dimethyl sulfoxide) to obtain a DMSO solution having a concentration of 50,000 ppm. To 3 mL of dissolved PDA medium was added 30 µL of each of the DMSO solution containing the fluorine-containing pyrazole compound prepared in Example 2 or 9 and DMSO, and the mixture obtained was stirred with a pipette, and placed in wells of a 6-well plate to solidify it (the fluorine-containing pyrazole compounds prepared in Examples 2 and 9 were placed in three wells, respectively). Rice blast fungi (Pyricularia oryzae, MAFF101511) grown in a PDA medium were cut out along with the medium using a biopsy punch of a 0.4 cm diameter, and these were each placed in the center of PDA medium containing the fluorine-containing pyrazole compound of Example 2 or 9 in the 6-well plate and allowed to grow at 27°C. The diameter of the fungal filaments was measured after 3 days.

Separately, rice blast fungi were also allowed to grow on three PDA media supplemented with DMSO in the same manner as above except that they were not treated with the fluorine-containing pyrazole compounds of Examples 2 and 9, and the diameter of fungal filaments was measured after 3 days. The average value of the three elongation lengths of fungal filaments thus obtained was defined as an "average of elongation lengths of fungal filaments without treatment."

A inhibition rate (%) of an elongation length of fungal filaments was calculated as follows: Inhibition rate = {(average of elongation lengths of fungal filaments without treatment - average of elongation lengths of fungal filaments with treatment by the fluorine-containing pyrazole compound prepared in Example 2 or 9)/average of elongation lengths of fungal filaments without treatment] × 100

**[Table 1]**

| Test compound | Inhibition rate (%) |
|---|---|
| Example 2 | 95 |
| Example 9 | 88 |

The inhibition rate by the fluorine-containing pyrazole compound prepared in Example 2 or 9 is shown in Table 1 above. As shown in Table 1 above, the fluorine-containing pyrazole compounds used in Examples 2 and 9, exhibited the high inhibition rate, from which these fluorine-containing pyrazole compounds can be said to have an excellent bactericidal activity against rice blast.

## Claims

1. A fluorine-containing pyrazole compound represented by the following general formula (1): 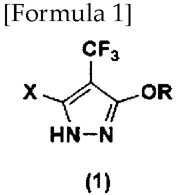
wherein R represents a hydrocarbon group having 1 to 12 carbon atoms,
X represents a heterocyclic group containing at least one heteroatom selected from the group consisting of a halogen atom, nitrogen and oxygen as a ring atom, -OA¹, or -NA¹A², and
A¹ and A² each independently represent a hydrogen atom or an organic group having 1 to 10 carbon atoms.

2. A method for producing a fluorine-containing pyrazole compound, comprising reacting a fluoroisobutylene derivative represented by the following general formula (2) with a compound represented by the following general formula (3) or a salt thereof to obtain a fluorine-containing pyrazole compound represented by the following general formula (4): 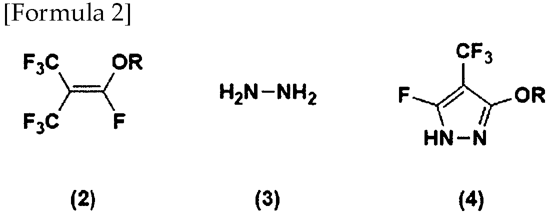 wherein R represents a hydrocarbon group having 1 to 12 carbon atoms.

3. A method for producing a fluorine-containing pyrazole compound, comprising reacting a fluoroisobutylene derivative represented by the following general formula (2) with a compound represented by the following general formula (3) or a salt thereof and a compound represented by the following general formula (5) to obtain a fluorine-containing pyrazole compound represented by the following general formula (1): 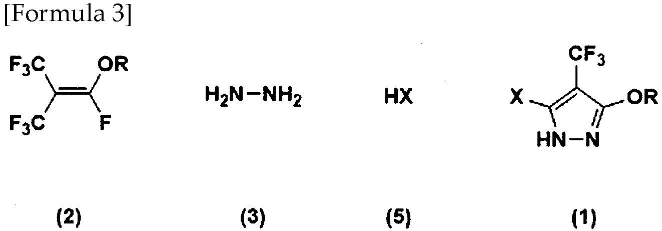
wherein R represents a hydrocarbon group having 1 to 12 carbon atoms,
X represents a heterocyclic group containing at least one heteroatom selected from the group consisting of a halogen atom, nitrogen and oxygen as a ring atom, -OA¹, or -NA¹A², and
A¹ and A² each independently represent a hydrogen atom or an organic group having 1 to 10 carbon atoms.

4. A method for producing a fluorine-containing pyrazole compound, comprising reacting a fluoroisobutane derivative represented by the following general formula (6) with a compound represented by the following general formula (3) or a salt thereof to obtain a fluorine-containing pyrazole compound represented by the following general formula (4): 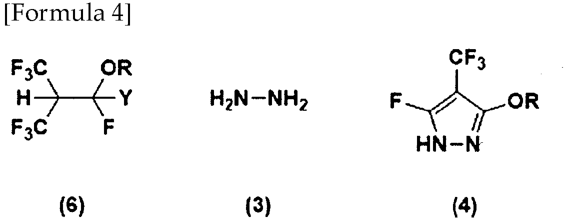
wherein R represents a hydrocarbon group having 1 to 12 carbon atoms,
Y represents a halogen atom, -OA³, -SOₘA³ where m is an integer of 0 to 3, or - NA³A⁴, and
A³ and A⁴ each independently represent a hydrogen atom or a hydrocarbon group having 1 to 10 carbon atoms.

5. A method for producing a fluorine-containing pyrazole compound, comprising reacting a fluoroisobutane derivative represented by the following general formula (6) with a compound represented by the following general formula (3) or a salt thereof and a compound represented by the following general formula (5) to obtain a fluorine-containing pyrazole compound represented by the following general formula (1): 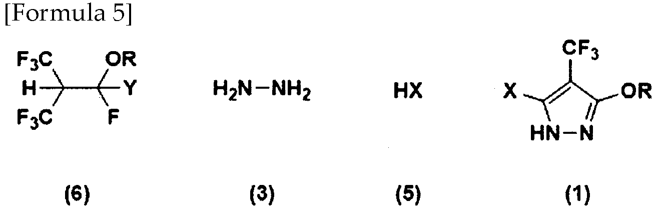
wherein R represents a hydrocarbon group having 1 to 12 carbon atoms,
X represents a heterocyclic group containing at least one heteroatom selected from the group consisting of a halogen atom, nitrogen and oxygen as a ring atom, -OA¹, or -NA¹A²,
A¹ and A² each independently represent a hydrogen atom or an organic group having 1 to 10 carbon atoms,
Y represents a halogen atom, -OA³, -SOₘA³ where m is an integer of 0 to 3, or - NA³A⁴, and
A³ and A⁴ each independently represent a hydrogen atom or a hydrocarbon group having 1 to 10 carbon atoms.
